# EUROPEAN PATENT SPECIFICATION

(11) **EP 0 627 075 B1**
(45) Date of publication and mention of the grant of the patent: **03.11.1999**
(21) Application number: 93904237.0
(22) Date of filing: 19.02.1993
(51) Int. Cl.: G01N 21/35, G01N 33/14

(54) **METHOD AND APPARATUS FOR ANALYSING LIQUIDS**
METHODE UND VORRICHTUNG ZUM ANALYSIEREN VON FLUESSIGKEITEN
PROCEDE ET APPAREIL D'ANALYSE DE LIQUIDES

(30) Priority: 19.02.1992 GB 9203461
(43) Date of publication of application: 07.12.1994
(73) Proprietor: PROCAL ANALYTICS LIMITED, Peterborough PE2 0HU (GB)
(72) Inventor: HUTCHINSON, Robin John, Peterborough PE6 8EW (GB); DAW, Christopher Blair, Oakham, Leicestershire LE15 9RZ (GB)
(74) Representative: Archer, Philip Bruce
(86) International application number: GB9300365
(87) International publication number: WO9317324

(56) References cited:
- EP-A- 0 243 139
- DE-A- 2 809 910
- FR-A- 670 046
- GB-A- 2 217 838

## Description

This invention relates to a method and apparatus for analysing liquids. A particular example of the application of the invention is to the analysis of the alcohol content of liquids such as beer, wine etc. The invention may also find application to analysis of the solute content of other solutions, such as ammonia in water.

The brewing industry requires accurate methods of measuring the alcohol content of beer produced, as duty is charged on beer on the basis of the alcohol content. Furthermore, the recent increase in the popularity of "low-alcohol" and "no-alcohol" beers has lead to a need for accurate control of the alcohol content at very low alcohol levels. Accordingly, the industry needs to be able to measure alcohol content at levels as low as, for example, 0.5%, and with an accuracy of 0.07%.

Previous proposals for the measurement of alcohol content in beer have included the use of near infra-red analysis apparatus employing transmission cells containing the beer to be analysed. Such methods are however mathematically complex and do not provide the required level of accuracy. They stretch the technology of near infra-red liquid analysis to its limits, and are almost certainly not adequate for the measurement of alcohol content in low-alcohol beers. A further factor is that this approach to the analysis is inevitably laboratory-based. Nevertheless, to-date it has been long accepted that this is the best that can be done.

The requirements of the brewing industry as discussed above include a need for apparatus and a method capable of providing substantially continuous monitoring of alcohol content, including measurement at the low levels of alcohol mentioned above, which are applicable to the production of beer on a continuous basis in an industrial plant.

Accordingly, we have identified a requirement for improvements in relation to the analysis of liquid solutions, particularly in relation to liquids such as beer, wine etc, offering improvements in relation to matters discussed above, or generally.

There is disclosed in FR-A-670,046 a method and apparatus for analysing a mixture of gases of vapours as defined in the pre-characterising portion of claim 1 hereof.

According to the invention there is provided a method and apparatus for analysis of liquid solutions as defined in the accompanying claims.

In a preferred embodiment, the method and apparatus comprises the step of vaporising the beer and measuring the alcohol content by the use of infra-red analysis apparatus operating in the vapour phase. As a result, the spectrum of alcohol is then distinct from the infra-red spectrum of water, and a dramatic improvement in the efficiency and accuracy of analysis is achieved. By the use of a vapour phase analysis as opposed to a liquid phase analysis as has been proposed previously, a significant improvement in performance is achieved, and the technique can be applied to analysis of the alcohol content of beer produced on a continuous basis, by arranging for sampling and vaporisation of the beer on a corresponding continuous basis.

In our published prior European patent application number EP 0 243 139 there is disclosed gas phase infra-red analysis apparatus capable of continuously measuring the concentration of up to three infra-red absorbing components in a gas phase stream. We hereby incorporate in the present application the entire disclosure of our prior European application.

In the preferred embodiment of the present invention, small volumes of beer are tapped from a main beer production pipeline. A small fixed rate of flow is defined by a pump, such as a peristaltic pump. This low rate of flow of beer is delivered to an evaporator maintained at approximately 110°C (the preferred range being from 100°C to 120°C), and in the evaporator both the water and the alcohol constituents of the beer boil. During the boiling, the increase in volume is more than one thousand times. This great increase is volume causes the vapour to force itself through a pipe kept at above 100°C into the infra-red analyser of the type disclosed in our prior European application. The analyser has a sample cell of total path length of one metre. The path is a folded path. The beer vapour passes through the sample cell which is kept at a fixed temperature above 100°C and passes to waste.

The analyser, being a multi-component analyser, can measure water vapour content, alcohol content and carbon dioxide content simultaneously. The wavelengths of measurement of each of these are distinct, with water vapour absorbing in the region of the spectrum between 2.5 and 3.0 microns and between 5 microns and 8 microns, alcohol absorbing mainly at 3.4 microns and carbon dioxide absorbing mainly at 4.28 microns. Simple measurement at these wavelengths is sufficient to make accurate measurements of alcohol and carbon dioxide. Cross sensitivities are easily corrected-for by means of a signal processor. To zero the apparatus, distilled water is fed into the evaporator in place of beer.

In the embodiment, simple flushing can be carried out to clear away residues left by previous liquids analysed, and likewise of cleaning fluids. It is noteworthy that the carbon dioxide content of beer can be measured simultaneously with the alcohol content.

In the preferred embodiment, continuous monitoring of alcohol content is provided by the use of sampling means connected to a beer delivery pipeline. The sampling means delivers a sample to the evaporator, which puts the sampled liquid into the vapour phase, after which the infra-red or other electromagnetic radiation analyser determines quantitatively the components of the sample. Signal processing apparatus analyses the signals generated by the analyser into readable data.

Thus, according to the invention, a method of analysing the alcohol content of beer, wine etc, and applicable to the analysis of other solutions, comprises the step of causing the sample to be vaporised. Such vaporisation may be achieved by heating, or indeed by pressure reduction, or by both.

The spectrum of alcohol in vapour-phase near infra-red analysis is distinct from the spectrum of water and other constituents such as carbon dioxide, whereby the vapour phase analysis is notably improved with respect to liquid phase analysis.

Also in the preferred embodiment, the analyser comprises datum means including a cell containing water vapour. Means is provided to prevent condensation of the water vapour during analysis. Typically, such means comprises heating means for the cell.

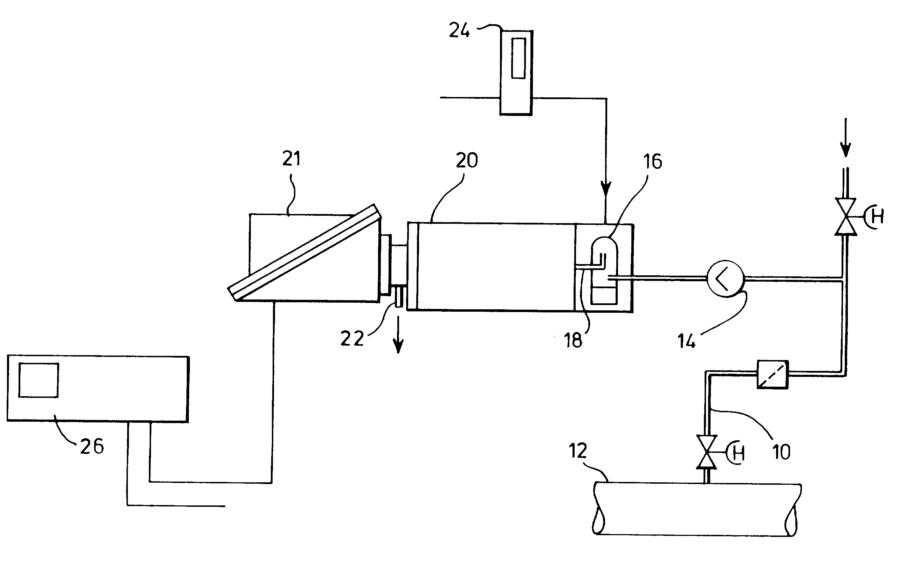

An embodiment of the invention will now be described by way of example with reference to the accompanying drawing which shows apparatus for the analysis of the alcohol content of beer.

In the apparatus of the drawing, the sequence of operations for analysing a sample of beer comprises the steps of removing a sample of beer through a tube 10 which taps into a main beer pipeline 12. The rate of flow of beer through tube 10 is regulated by a pump 14. The tube 10 feeds the sample into an evaporator 16 which is maintained at a temperature in the range of 105 to 115°C, preferably 110°C. The evaporator 16 is connected to a gas analysis cell 20 by means of a connecting tube 18.

Once in the evaporator 16, the sample boils and vaporises. The vaporisation causes an increase in the volume of the sample of more than one thousand-fold. This increase in volume causes the vapour to force itself into connecting tube 18 and hence into cell 20. The connecting tube 18 is maintained at a temperature above 100°C, and likewise the cell 20 of analyser 21, so as to maintain the beer in the vapour phase and prevent condensation. Temperature maintenance is by means of a temperature controller 24 controlling a heat source (not shown).

During passage of the vapour through the sample cell 20, analyser 21 carries out infra-red analysis of the vapour phase constituents of the beer namely alcohol, carbon dioxide and water. Signal processing apparatus 26 is connected to analyser 21 and analyses signals generated thereby so as to produce readable data. The function of analyser 21 and signal processing apparatus 26 is substantially as disclosed in our prior European patent application.

Analyser 21 comprises datum means (not shown) to intercept the radiation and to transmit it to a sensor at a reference or datum value. Such datum means comprises one or more gas cells containing a relatively high concentration of gas to be analysed. In the present application, at least one of the cells of the datum means contains water vapour or steam. The temperature of the apparatus maintains the steam in the vapour phase.

Interestingly, the above embodiment provides a method and apparatus for measuring the alcohol content of wines and beers providing greater accuracy of results, as a result of the vapour phase analysis technique, and without the need to take samples for laboratory analysis. Continuous monitoring is conveniently carried out.

The method and apparatus described in the above embodiment can be used for the analysis of dissolved components in any suitable liquid, where the constituents of the solution can be vaporised and detected by an electromagnetic radiation analysis device.

Modifications which could be made in the above embodiment while remaining within the scope of the invention include the use of alternative radiation frequencies such as ultra-violet, with appropriate changes in the radiation source and the radiation sensor. A further modification comprises the provision of modified means for maintenance of the liquids in the vapour phase, including the use of reduced pressure and different ranges of temperatures.

The embodiment provides significant advantages over currently available chromatography techniques in view of the maintenance requirements of such apparatus and their cycle times.

## Claims

1. A method of analysis of liquid samples comprising :
a) providing a radiation source and employing same to transmit radiation through a sample to be analysed;
b) providing a radiation sensor and employing same to sense radiation from said source, transmitted through said sample; and
c) providing signal processing apparatus (26) and employing same to analyse signals generated by said sensor in response to radiation transmitted to determine the content of the sample;
d) the step of treating said liquid sample to cause it to enter the vapour phase prior to transmission of said radiation through the sample; and
characterised in that
e) said liquid samples are tapped from a main liquid production pipeline through a tube (10) for continuous monitoring of the content of said liquid.

2. A method according to claim 1 characterised by providing datum means positionable to intercept radiation from said source and employing said datum means to transmit the radiation to said sensor at a reference or datum value.

3. A method according to claim 2 characterised by transmitting said radiation through a cell containing water vapour or steam.

4. A method according to claim 3 characterised by the step of maintaining said cell at a temperature and/or pressure such as to maintain said steam in the vapour phase.

5. Apparatus for the analysis of liquid samples comprising:
a) a gas phase analysis apparatus (20) comprising a radiation source and a radiation sensor (21);
b) an evaporator (16) for vaporising a liquid sample to be analysed and for subjecting the resultant vapour phase to radiation from said analyser ; characterised by
c) a tube (10) feeding said liquid sample to said evaporator (16) from a main liquid pipeline (12), to monitor the content of said samples.

6. Apparatus according to claim 5 characterised by means for maintaining said liquid sample in the vapour phase.

7. Apparatus according to claim 6 characterised by said means for maintaining said sample in the vapour phase comprising heating means.

## Patentansprüche

1. Methode zum Analysieren von flüssigen Proben mit
a) einer Strahlungsquelle zum Transmittieren einer Strahlung durch eine zu analysierende Probe,
b) einem Strahlungsfühler zum Nachweisen der durch die Probe transmittierten Strahlung der Quelle,
c) einer Signalverarbeitungsanlage (26) zum Analysieren des von dem Fühler entsprechend der transmittierten Strahlung erzeugten Signals, um den Inhalt der Probe zu bestimmen, und
d) dem Schritt, die flüssige Probe vor der Transmission der Strahlung durch die Probe in die Gasphase zu überführen,
dadurch **gekennzeichnet**, daß
e) die flüssigen Proben durch eine Rohrleitung (10) zum kontinuierlichen Überwachen des Inhalts der Flüssigkeit aus einer Flüssigkeitshauptproduktionsrohrleitung entnommen werden.

2. Verfahren nach Anspruch 1, dadurch **gekennzeichnet**, daß Mittel zum Aufnehmen von Referenzdaten vorgesehen sind, die den Strahlengang der Quelle unterbrechen und die die Strahlung zu dem Fühler als Referenz- oder Bezugswert übertragen.

3. Verfahren nach Anspruch 2, dadurch **gekennzeichnet**, daß die Strahlung durch eine Zelle transmittiert wird, die Wasserdampf enthält.

4. Verfahren nach Anspruch 3, **gekennzeichnet** durch den Schritt, die Zelle bei einer derartigen Temperatur und/oder einem derartigen Druck zu betreiben, daß der Dampf in die Gasphase übertritt.

5. Vorrichtung zum Analysieren von flüssigen Proben, mit
a) einer Gasphasenanalysenanordnung (20) mit einer Strahlungsquelle und einem Strahlungsfühler (21),
b) einem Verdampfer (16) zum Verdampfen einer zu analysierenden flüssigen Probe und zum Beaufschlagen der resultierenden Gasphase mit Strahlung von dem Analysator,
dadurch **gekennzeichnet**, daß
c) eine Rohrleitung (10) den Verdampfer (16) mit der flüssigen Probe aus einer Hauptflüssigkeitsleitung (12) zum Überwachen des Inhalts der Proben speist.

6. Vorrichtung nach Anspruch 5, dadurch **gekennzeichnet**, daß Mittel zum Überleiten der flüssigen Probe in die Gasphase vorgesehen sind.

7. Vorrichtung nach Anspruch 6, dadurch **gekennzeichnet**, daß die Mittel zum Überleiten der Probe in die Gasphase Heizmittel haben.

## Revendications

1. Procédé d'analyse d'échantillons de liquide, comprenant :
(a) la préparation d'une source de rayonnement et l'utilisation de cette source pour transmettre un rayonnement à travers un échantillon à analyser ;
(b) la préparation d'un détecteur de rayonnement et l'utilisation de ce détecteur pour détecter le rayonnement de ladite source transmis à travers ledit échantillon ;
(c) la préparation d'un appareil de traitement de signal (26) et l'utilisation de cet appareil pour analyser les signaux engendrés par ledit détecteur en réponse au rayonnement transmis, afin de déterminer le contenu de l'échantillon ;
(d) l'étape de traitement dudit échantillon de liquide pour provoquer son passage en phase vapeur avant la transmission dudit rayonnement à travers l'échantillon ; caractérisé en ce que ;
(e) lesdits échantillons de liquide sont prélevés à partir d'une canalisation de production de liquide principale, par l'intermédiaire d'un tube (10), pour surveillance continue du contenu dudit liquide.

2. Procédé selon la revendication 1, caractérisé en ce qu'il comprend la préparation d'un moyen de référence positionnable de manière à intercepter le rayonnement de ladite source, et l'utilisation dudit moyen de référence pour transmettre le rayonnement audit détecteur, à une valeur de référence ou de repère.

3. Procédé selon la revendication 2, caractérisé par la transmission dudit rayonnement à travers une cellule contenant de la vapeur d'eau.

4. Procédé selon la revendication 3, caractérisé par l'étape de maintien de ladite cellule à une température et/ou une pression telles que ladite vapeur d'eau reste dans la phase vapeur.

5. Appareil pour l'analyse d'échantillons de liquide comprenant :
(a) un appareil d'analyse en phase gazeuse (20) comportant une source de rayonnement et un détecteur de rayonnement (21) ;
(b) un évaporateur (16) pour évaporer un échantillon de liquide à analyser et pour soumettre la phase vapeur résultante à un rayonnement dudit analyseur ;
caractérisé par :
(c) un tube (10) amenant ledit échantillon de liquide audit évaporateur (16) à partir d'une canalisation de liquide principale (12), afin de surveiller le contenu desdits échantillons.

6. Appareil selon la revendication 5, caractérisé par des moyens de maintien dudit échantillon de liquide dans la phase vapeur.

7. Appareil selon la revendication 6, caractérisé en ce que lesdits moyens de maintien dudit échantillon dans la phase vapeur comprennent des moyens de chauffage.
